(19) Europäisches Patentamt / European Patent Office / Office européen des brevets

(11) **EP 3 068 455 B1**

(12) **EUROPÄISCHE PATENTSCHRIFT**

(45) Veröffentlichungstag und Bekanntmachung des Hinweises auf die Patenterteilung:
**18.10.2017 Patentblatt 2017/42**

(21) Anmeldenummer: **14798795.2**

(22) Anmeldetag: **12.11.2014**

(51) Int Cl.:
*A61M 1/00* (2006.01)    *A61F 13/00* (2006.01)
*B01D 63/08* (2006.01)    *B01D 63/00* (2006.01)
*B01D 69/00* (2006.01)    *A61M 27/00* (2006.01)

(86) Internationale Anmeldenummer:
**PCT/EP2014/074311**

(87) Internationale Veröffentlichungsnummer:
**WO 2015/071279 (21.05.2015 Gazette 2015/20)**

(54) **TASCHENFÖRMIGES WUNDAUFLAGESYSTEM**

BAG-SHAPED WOUND DRESSING SYSTEM

TROUSSE DE PANSEMENTS EN FORME DE POCHE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.11.2013 EP 13192646**

(43) Veröffentlichungstag der Anmeldung:
**21.09.2016 Patentblatt 2016/38**

(73) Patentinhaber: **3M Innovative Properties Company**
**St. Paul, MN 55133-3427 (US)**

(72) Erfinder:
• **MEIXNER, Carsten**
**42499 Hückeswagen (DE)**
• **KORBI, Haythem**
**42289 Wuppertal (DE)**

(74) Vertreter: **Schröder, Richard et al**
**3M Belgium bvba/sprl**
**Hermeslaan 7**
**1831 Diegem (BE)**

(56) Entgegenhaltungen:
**WO-A1-2010/037092    DE-B3-102006 042 732**
**US-A- 5 549 584**

EP 3 068 455 B1

**Beschreibung**

Die Erfindung betrifft ein Wundauflagesystem umfassend mindestens ein mattenförmiges Kapillarmembransystem zum Einbringen in eine Wunde bzw. zum Aufbringen auf eine Hautwunde und unter einen Wundverband.

Die moderne Wundversorgung verfolgt das Ziel, bei der Wundversorgung ein feuchtes Wundmilieu zu schaffen, das bei der Heilung ablaufende Prozesse fördert. Je nach Heilungsphase müssen moderne, aktive Wundauflagen deshalb in der Lage sein, die Wunde feucht zu halten und/oder große Mengen an Exsudat abzuführen. In der Wundbehandlung kommt es darauf an, für einen verbesserten Flüssigkeits-/Stoffaustausch, für das Einbringen von Faktoren/Medikamenten und/oder für eine verbesserte Flüssigkeits-/Sekrets- und/oder Stoffentfernung in der/in die Wunde bzw. aus der Wunde zu sorgen. Anwendungen schließen die Nutzung solcher Wundauflagesysteme in einer Weichteilwunde, in einer abdominellen Wunde und auf einer Hautwunde ein.

Ein Verfahren und eine Vorrichtung zur Entfernung von Sekret bzw. Exsudat aus Wunden ist kommerziell bekannt als V.A.C.® Therapy System (Fa. KCI, USA). Bei diesem System wird für ein alternierendes Einbringen von Flüssigkeit in die Wunde und ein nachfolgendes, also auch alternierendes und damit nicht kontinuierliches Ausführen von Flüssigkeit aus der Wunde gesorgt. Ein in die Wunde eingebrachtes Schaumstoffmaterial, das bei Unterdruck Kräfte auf die Wunde ausübt, soll bei diesem System die Wundheilung begünstigen.

In der DE 10 2006 042 732 wird ein Kapillarmembransystem zur Wundbehandlung beschrieben, bei dem die Wunde über eine Hohlfasermembrananordnung aus bis zu 1000 Hohlfasern mit mindestens einer gemeinsamen Zu- und mindestens einer gemeinsamen Ableitung im Sinne der Durchströmung eines Kapillarbettes perfundiert und versorgt werden und eine Antibiotika- sowie Wachstumsfaktorenperfusion ermöglicht werden soll. Dabei soll eine gleichmäßige Stoffverteilung unter kontinuierlicher Perfusion auch unter Erzeugung eines moderaten Unterdruckes ermöglicht werden. Die Vorrichtung soll des Weiteren in der Lage sein, parallel bei der Wundbehandlung Zellen einzubringen, um damit das Behandlungsergebnis zu verbessern .

Wenngleich sich bereits mit den in der DE 10 2006 042 732 beschriebenen Kapillarmembransystemen Fortschritte bei der Wundbehandlung erzielen lassen, besteht weiterhin Bedarf an verbesserten Wundbehandlungssystemen, mit denen zum einen die bei der Wundbehandlung notwendigen Vorgänge, wie Spülen und Desinfizieren, ohne Entfernen des Verbandes durchgeführt werden können, die erforderlichenfalls eine Ernährung, Temperierung, Oxygenierung, pH-Regulation, Elektrolytaustausch und/oder Detoxifikation oder auch eine Wachstumsfaktorenversorgung oder eine Versorgung mit Antibiotika ermöglichen und die eine einfache und sichere Handhabung erlauben.

Es ist Aufgabe der vorliegenden Erfindung, ein derartiges Wundbehandlungssystem zur Verfügung zu stellen.

Die Aufgabe wird durch ein Wundauflagesystem zum Einbringen in eine Wunde gelöst, welches mindestens ein flächenförmig ausgebildetes Kapillarmembransystem umfasst und welches dadurch gekennzeichnet ist, dass das mindestens eine flächenförmig ausgebildete Kapillarmembransystem in einer taschenförmigen Wundauflage angeordnet ist, die an ihrem äußeren Rand geschlossen ist und eine Oberseite, eine Unterseite und ein Tascheninneres aufweist,

- wobei die Unterseite und die Oberseite jeweils aus einem flächigen Material ausgebildet sind und die Unterseite permeabel für Fluide ist,
- wobei das mindestens eine flächenförmig ausgebildete Kapillarmembransystem im Tascheninneren angeordnet und mit mindestens einer Versorgungsleitung verbunden ist, so dass durch die Versorgungsleitung und das mindestens eine Kapillarmembransystem Flüssigkeiten, Medien, Gase und/oder andere Stoffe durchleitbar sind, und
- wobei die mindestens eine Versorgungsleitung außerhalb der taschenförmigen Wundauflage mit einer Einheit zur Versorgung bzw. Entsorgung verbindbar ist.

Das Wundauflagesystem wird so in eine zu behandelnde Wunde eingebracht, dass die Unterseite der taschenförmigen Wundauflage auf der Wunde aufliegt. Über das mindestens eine flächenförmig ausgebildete Kapillarmembransystem kann der Wunde beispielsweise Flüssigkeit z.B. in Form einer Nährlösung zugeführt werden, die sich nach Austritt aus den Kapillarmembranen in der Tasche verteilt und über die semipermeable Taschenunterseite an die Wunde abgegeben wird. Über die mindestens eine Versorgungsleitung ist dann das Kapillarmembransystem z.B. über eine Pumpe mit einer Versorgungseinheit für die Flüssigkeit, z.B. einem Vorratsbehälter für die Nährlösung verbunden.

Das mindestens eine Kapillarmembransystem kann auch zur Absaugung von Exsudat aus der Wunde eingesetzt werden. In diesem Fall ist die Versorgungseinheit eine Unterdruckeinheit, die mit dem Kapillarmembransystem über mindestens eine Versorgungsleitung verbunden ist.

Die Versorgung der Wunde mit einer Flüssigkeit und Absaugung von Exsudat aus der Wunde kann über das mindestens eine Kapillarmembransystem auch intermittierend durchgeführt werden, indem die mindestens eine Versorgungsleitung über beispielsweise ein T-Stück über eine erste Teilleitung mit einer Flüssigkeitsvorlage und über eine zweite Teilleitung mit einer Unterdruckeinheit verbunden ist. Mittels ansteuerbarer Absperrventile kann über vorgegebene Zeitintervalle entweder eine Versorgung mit Flüssigkeit oder eine Absaugung von Exsudat erfolgen.

In einer Ausführungsform sind die Kapillarmembranen mindestens eines Kapillarmembransystems für die Zuführung bzw. Abführung von flüssigen Medien ausgelegt. Um in diesem Fall eine gleichmäßige Ver- bzw. Entsorgung der Wunde zu gewährleisten, weisen dann die Kapillarmembranen bevorzugt eine hohe Durchlässigkeit für Flüssigkeiten auf. Vorzugsweise liegt dabei der Transmembranfluss für Wasser der Kapillarmembranen im Bereich von 0,01 bis 50 ml/(min·cm$^2$·bar) auf.

In einer weiteren bevorzugten Ausführungsform umfasst das Wundauflagesystem mindestens zwei flächenförmig ausgebildete Kapillarmembransysteme, wobei eines der Kapillarmembransysteme für den Gastransfer geeignete Kapillarmembranen aufweist, z.B. Membranen für die Oxygenation, wie sie etwa in der EP-A-1 144 096, der EP-A-0 299 381 oder der DE-A-28 33 493 offenbart sind. Über ein derartiges Kapillarmembransystem kann beispielsweise eine Oxygenierung, d.h. eine Versorgung der zu behandelnden Wunde mit Sauerstoff erfolgen.

Unter dem mindestens einen flächenförmig ausgebildeten Kapillarmembransystem wird eine Anordnung aus mindestens einer Kapillarmembran verstanden, die sich flächig im Tascheninneren erstreckt. Die Dimensionen des mindestens einen flächenförmig ausgebildeten Kapillarmembransystems ergeben sich aus dessen äußeren Abmessungen in der flächigen Erstreckung. Vorzugsweise füllt das mindestens eine Kapillarmembransystem hinsichtlich seiner flächigen Erstreckung das Tascheninnere der taschenförmigen Wundauflage in dessen flächiger Erstreckung mindestens zu 20 % und besonders bevorzugt mindestens zu 50 % aus. Von besonderem Vorteil ist es, wenn das mindestens eine Kapillarmembransystem hinsichtlich seiner flächigen Erstreckung das Tascheninnere der taschenförmigen Wundauflage in dessen flächiger Erstreckung mindestens zu 70 % ausfüllt, wobei auch Füllgrade im Bereich von 90 % realisiert werden können. Dabei ist es von Vorteil, wenn das flächenförmig ausgebildete Kapillarmembransystem in der taschenförmigen Wundauflage mittig angeordnet ist.

Hierbei kann das mindestens eine flächenförmig ausgebildete Kapillarmembransystem aus einer einzelnen Kapillarmembran bestehen, die mäanderförmig im Tascheninneren angeordnet ist. Bei dieser Ausführungsform ist mindestens eines der Enden der mäanderförmigen Kapillarmembran offen und mit einer Versorgungsleitung verbunden. Das mindestens eine Kapillarmembransystem kann jedoch auch mehrere mäanderförmig angeordnete Kapillarmembranen umfassen, die zusammen mit ihren Enden in eine gemeinsame Versorgungsleitung münden.

Die Anzahl der Kapillarmembranen im mindestens einen flächenförmig ausgebildeten Kapillarmembransystem hängt natürlich in erster Linie von der Größe des Wundversorgungssystems und damit von der Größe der taschenförmigen Wundauflage ab, die wiederum auf die Größe der damit zu behandelnden Wunde abzustimmen ist. Das mindestens eine flächenförmig ausgebildete Kapillarmembransystem kann daher aus etwa 10 bis zu mehreren hundert oder tausend zueinander parallel angeordneten Kapillarmembranen aufgebaut sein.

Die zueinander parallel angeordneten Kapillarmembranen sind an mindestens einem ihrer Enden so an ihrem äußeren Umfang fluiddicht in der Wand der mindestens einen Versorgungsleitung eingebettet, dass zwischen dem Lumen der Versorgungsleitung und dem Lumen der Kapillarmembranen eine Fluidverbindung besteht und durch die Versorgungsleitung und das mindestens eine Kapillarmembransystem Flüssigkeiten, Medien, Gase und/oder andere Stoffe durchleitbar sind. Vorzugsweise ist die mindestens eine Versorgungsleitung an ihrem einen Ende offen und mit einer Versorgungseinheit oder einer Einheit zur Entsorgung verbindbar, während das andere Ende der mindestens einen Versorgungsleitung geschlossen ist. Die Einbettung kann beispielsweise mit einem härtbaren Silikonmaterial, mit einem Polyurethan- oder einem Epoxidharz erfolgen. Bevorzugt werden wegen ihrer besseren Flexibilität härtbare Silikonmaterialien eingesetzt. Im Falle, dass die Kapillarmembranen nur mit einem ihrer Enden in einer Versorgungsleitung eingebettet sind, ist das andere, gegenüberliegende Ende der Kapillarmembranen geschlossen, beispielsweise durch Verschweißen oder Verkleben. Die Kapillarmembranen können auch an ihren beiden Enden offen und mit diesen beiden Enden auf einer Seite der Anordnung in eine Versorgungsleitung eingebettet sein, wobei die Kapillarmembranen dann an ihrem freien Ende U-förmig ausgebildet sind und dadurch dort geschlossen sind. In diesen Fällen werden die Kapillarmembranen im Dead-end Modus betrieben.

Insbesondere bei breiteren Wundauflagesystemen ist eine Ausführungsform des mindestens einen Kapillarmembransystems mit parallel zueinander angeordneten Kapillarmembranen von Vorteil, bei dem die Kapillarmembranen an ihren beiden Enden offen und in jeweils eine Versorgungsleitung eingebettet sind, wobei sich die Versorgungsleitungen sich dann bevorzugt an gegenüberliegenden Seiten der taschenförmigen Wundauflage befinden. Auch in diesem Fall ist die Einbettung so ausgeführt, dass die Kapillarmembranen an ihrem äußeren Umfang fluiddicht eingebettet sind und zwischen dem Lumen der jeweiligen Versorgungsleitung und dem Lumen der Kapillarmembranen eine Fluidverbindung besteht. Eine derartige Ausführungsform mit zwei Versorgungsleitungen erlaubt eine Versorgung und/oder Entsorgung über das mindestens eine Kapillarmembransystem im crossflow Modus. Auch mit Blick auf eine gute Homogenität der Versorgung bzw. Entsorgung über der Fläche der Wunde kann insbesondere bei breiteren Matten bzw. Wundauflagen die Ausführung des mindestens einen Kapillarmembransystems mit zwei Versorgungsleitungen zweckmäßig sein.

Der Durchmesser der mindestens einen Versorgungsleitung richtet sich in erster Linie nach dem Außendurchmesser der in sie eingebetteten Kapillarmembranen. Daher weist die mindestens eine Versorgungsleitung bevorzugt einen Innendurchmesser im Bereich von 0,1 bis 10 mm auf. Ebenso ist bevorzugt, wenn die Wandstärke im Bereich von 0,1 bis 5 mm liegt. Im Falle der Verwendung einer Versorgungsleitung mit nicht-kreisförmigem Querschnitt wird als Innendurchmesser der äquivalente Durchmesser $d = 4A/U$ des Innenquerschnitts angesetzt mit A als der Fläche des Innenquerschnitts und U als dessen Umfang. Beispielsweise kann die Versorgungsleitung auch einen ovalen, oder näherungsweise quadratischen oder rechteckigen Innenquerschnitt aufweisen. Für die mindestens eine Versorgungsleitung hat sich beispielsweise ein Silikonschlauch als geeignet erwiesen, durch dessen Wand die Kapillarmembranenden hindurchtreten und in der sie eingeklebt sind. Vorzugsweise ist die mindestens eine gemeinsame Versorgungsleitung ein flexibler Silikonschlauch. Die Einbettung bzw. das Einkleben in die Wand der Versorgungsleitung kann mittels üblicher Kleber wie z.B. mittels härtbarer Silikonmaterialien, Polyurethanharzen oder einem Epoxidharzen erfolgen. In einer Ausführungsform des Wundauflagesystems kann die Verbindung des mindestens einen flächenförmig ausgebildeten Kapillarmembransystems mit der mindestens einen Versorgungsleitung außerhalb der taschenförmigen Wundauflage auf der Oberseite angeordnet sein und das mindestens eine flächenförmig ausgebildete Kapillarmembransystem zur Verbindung mit der mindestens einen Versorgungsleitung über eine fluiddicht angepasste Durchtrittsöffnung aus der taschenförmigen Wundauflage herausführen. Die Durchtrittsöffnung kann auch z.B. mittels eines Silikonmaterials abgedichtet sein.

In einer weiteren Ausführungsform des Wundauflagesystems kann sich die Verbindung des mindestens einen flächenförmig ausgebildeten Kapillarmembransystems mit der mindestens einen Versorgungsleitung im Tascheninneren befinden und die mindestens eine Versorgungsleitung über eine an ihren äußeren Querschnitt fluiddicht angepasste Durchtrittsöffnung aus der taschenförmigen Wundauflage herausführen.

In einer vorteilhaften Ausführungsform kann das Kapillarmembransystem in Form einer Kapillarmembranmatte ausgebildet sein, in der mehrere zueinander parallele Kapillarmembranen mittels zueinander beabstandeter und zueinander parallel verlaufender Verbindungselemente miteinander verbunden und durch die Verbindungselemente zueinander auf Abstand gehalten sind. Dabei können die Verbindungselemente quer zu den parallel zueinander angeordneten Kapillarmembranen verlaufen oder auch unter einem anderen Winkel. Bei den Verbindungselementen kann es sich um Klebestreifen handeln oder beispielsweise auch um strangförmige Elemente aus einem Silikonmaterial. In einer bevorzugten Ausführungsform sind die Kapillarmembranen mittels garnförmiger Verbindungselemente zu einer Matte verbunden. Besonders bevorzugt handelt es sich bei den Verbindungselementen um textile Multifilamentgarne. Besonders bewährt haben sich als textile Multifilamentgarne multifile Polyestergarne, Polypropylengarne oder Polytetrafluorethylengarne.

Bei der Kapillarmembranmatte kann es sich in einer bevorzugten Ausführungsform um eine Wirkmatte handeln. Bei solchen Wirkmatten sind die Kapillarmembranen und die Verbindungsfäden miteinander verwirkt und die Kapillarmembranen verlaufen quer zur Erstreckungsrichtung der Kapillarmembranmatte. Die Länge der Kapillarmembranen ist durch die Mattenbreite bestimmt. In einer weiteren bevorzugten Ausführungsform kann es sich bei der Kapillarmembranmatte um eine Webmatte handeln. Bei solchen Webmatten sind die Kapillarmembranen und die Verbindungsfäden miteinander verwoben. Die Kapillarmembranen verlaufen dabei in Erstreckungsrichtung oder Laufrichtung der Kapillarmembranmatte und die textilen Fäden quer dazu. Kapillarmembranwirkmatten und -webmatten sowie Möglichkeiten ihrer Herstellung werden beispielsweise in der DE 38 39 567, der DE 43 08 850 und in der EP 0 442 147 beschrieben. Insbesondere mittels der Wirktechnologie lassen sich auf einfache Weise Matten herstellen, bei denen die Kapillarmembranen an ihrem freien Ende U-förmig ausgebildet und dort verschlossen sind. Derartige Matten können durch mäanderförmige Ablage einer Kapillarmembran in zueinander parallele Stränge, die durch die Wirkfäden miteinander verbunden sind, hergestellt werden. Dabei werden nach Fertigstellung der Wirkmatte die U-förmig ausgebildeten Enden an mindestens einer Seite der Wirkmatte abgetrennt und die dabei entstehenden offenen Enden der Kapillarmembranen dann einer Versorgungsleitung eingebettet. Im Falle, dass die U-förmig ausgebildeten Enden an beiden Seite der Wirkmatte abgetrennt werden, werden die entstehenden gegenüberliegenden offenen Enden jeweils in Versorgungsleitungen eingebettet.

Als Materialien für die Kapillarmembranen kommen grundsätzlich alle im Stand der Technik bekannten organischen Polymere in Frage, die zur Ausbildung von Kapillarmembranen geeignet sind, wobei diese Polymere eine gute Biokompatibilität ausweisen müssen. Darüber hinaus ist es auch erforderlich, dass das Membranpolymer eine Sterilisation des Wundauflagesystems beispielsweise über Dampfsterilisation, Sterilisation mittels $\gamma$-Strahlung oder Sterilisation mittels Ethylenoxid erlaubt. Dabei können die organischen Polymere natürliche Polymere sein oder Polymere, die auf synthetischen Wege hergestellt wurden. Natürliche Polymere sind insbesondere solche auf Basis von zellulosischen Polymeren, was Polymere, die sog. polymeranalogen Reaktionen unterzogen worden sind, ebenfalls umfasst. Beispiele für Polymere auf Basis von Zellulose sind solche aus regenerierter Zellulose, Zelluloseazetat oder modifizierter Zellulose wie z.B. Zelluloseester, Zelluloseäther, mit Benzylgruppen modifizierte Zellulose (Benzylzellulose) oder mit Diethylaminoethyl modifizierte Zellulose oder Mischungen dieser zellulosischen Polymere. Des Weiteren können auch Polymere auf der Basis von Chitin, bzw. Chitosan zum Einsatz kommen.

Als auf synthetischem Wege hergestellte Polymere, d.h. als synthetische Polymere können solche verwendet werden, die aus Polyolefinen, Polyamiden, Polyacrylnitrilen, Polycarbonaten, Polyestern oder Sulfonpolymeren sowie daraus gewonnen Modifikationen, Blends, Mischungen oder Copolymere dieser Polymere bestehen. Vorzugsweise werden solche verwendet, die auf Sulfonpolymeren, wie insbesondere Polysulfon oder Polyethersulfon, basieren. Den synthetischen Polymeren können weitere Polymere wie z.B. Polyethylenoxid, Polyhydroxyether, Polyethylenglykol, Polyvinylalkohol oder Polycaprolacton als Zusatzstoffe beigemischt werden. Die Kapillarmembranen können darüber hinaus noch eine Beschichtung mit einem Additiv aufweisen. Bevorzugt enthalten solche Kapillarmembranen ein Hydrophilierungsmittel, z.B. Polyvinylpyrrolidon oder auch hydrophile Modifikationen dieser Polymere.

Die Kapillarmembranen können mit Blick auf bestimmte Anwendungen z.B. über Ankopplung funktioneller Gruppen modifiziert sein oder beispielsweise mit Heparin oder einem Antibiotikum oder mehreren Antibiotika beschichtet sein.

Das Wundauflagesystem umfasst eine taschenförmig ausgebildete Wundauflage, in deren Tascheninneren das mindestens eine Kapillarmembransystem angeordnet ist. Dabei kann die taschenförmige Wundauflage beliebige Konturen aufweisen. Vorzugsweise ist die Kontur jedoch rund, oval, quadratisch oder rechteckig, und Unterseite und Oberseite der taschenförmigen Wundauflage sind am äußeren Rand bzw. an den äußeren Kanten der Wundauflage beispielsweise durch Verschweißen oder Verkleben miteinander verbunden. Zur Verklebung sind u.a. Silikonstreifen geeignet, die ausgehärtet werden. Bei rechteckigen oder bei quadratischen taschenförmigen Wundauflagen weist das darin angeordnete flächenförmig ausgebildete Kapillarmembransystem vorzugsweise ebenfalls eine rechteckige oder quadratische Kontur auf. Bei runden oder ovalen taschenförmigen Wundauflagen ist das darin befindliche mindestens eine flächenförmig ausgebildete Kapillarmembransystem zweckmäßigerweise ebenfalls quadratisch oder rechteckig ausgebildet, wobei hinsichtlich der Abmessungen ebenfalls die zuvor genannten Dimensionen gelten. Es kann jedoch auch an die Kontur der taschenförmigen Wundauflage angepasst sein, beispielsweise durch entsprechend angepasstes Abschweißen der nicht eingebetteten Enden der Kapillarmembranen bei Kapillarmembransystemen mit nur einer Versorgungsleitung, so dass sich an dieser Kante des flächenförmig ausgebildeten Kapillarmembransystems eine bogenförmige Kontur ergibt.

Wie bereits ausgeführt wurde, hängt natürlich die Größe der taschenförmigen Wundauflage in erster Linie von der Größe der damit zu behandelnden Wunde ab. Die Größe der taschenförmigen Wundauflage in ihren flächigen Erstreckungsrichtungen kann dabei je nach Anwendung im Bereich von ca.1 cm bis hin zu ca. 1 m oder mehr betragen.

Die Unterseite der taschenförmigen Wundauflage ist gegenüber Fluiden durchlässig, d.h. permeabel. Dabei kann die Unterseite beispielsweise aus einem vliesförmigen, flächigen Material, einem gitterförmigen oder netzartigen Material, einer perforierten Folie oder einer semipermeablen mikroporösen Flachmembran bestehen. In einer vorteilhaften Ausführungsform besteht die Unterseite aus einem vliesförmigen, flächigen Material oder einer semipermeablen mikroporösen Flachmembran. Die Unterseite weist bevorzugt eine Permeabilität für Wasser von mindestens 0,01 ml/(min·cm$^2$·bar) und besonders bevorzugt von mindestens 10 ml/(min·cm$^2$·bar) auf. Bestens bewährt hat sich eine Unterseite mit einer Permeabilität für Wasser von mindestens 500 ml/(min·cm$^2$·bar).

Für Anwendungen des Wundauflagesystems, bei denen die Wunde über das Wundauflagesystem nicht nur mit Flüssigkeit versorgt wird, sondern auch eine Entsorgung, d.h. Abführung von Flüssigkeiten aus der Wunde und insbesondere eine Entsorgung von Exsudat erfolgen soll, ist es von Vorteil, wenn in der Unterseite Öffnungen vorhanden sind, wobei die Öffnungen vorzugsweise einen Durchmesser von mindestens 100 $\mu$m aufweisen. Dabei sind Durchmesser der Öffnungen von höchstens 10 mm bevorzugt und von höchstens 5 mm besonders bevorzugt. Im Falle, dass die Unterseite aus einer semipermeablen mikroporösen Flachmembran besteht, weist diese in einer vorteilhaften Ausführungsform zusätzlich Öffnungen z.B. in Form von Perforationen auf. Im Falle, dass die Öffnungen eine nicht-kreisförmige Kontur aufweisen, wird als Durchmesser der äquivalente Durchmesser D=4A/U der Öffnung angesetzt mit A als der Fläche der jeweiligen Öffnung und U als deren Umfang. Die Öffnungen können regelmäßig oder unregelmäßig über die Fläche der Unterseite verteilt sein, wobei eine regelmäßige, homogene Verteilung bevorzugt ist. Dabei kann der Abstand zwischen den Öffnungen im Bereich von 1 bis 20 mm liegen, gemessen von äußeren Rand der Öffnungen.

Unter- und die Oberseite der taschenförmigen Wundauflage können aus gleichen oder unterschiedlichen Materialien bestehen. Während die Unterseite jedoch stets gegenüber Flüssigkeiten durchlässig ist, ist die Oberseite bevorzugt aus einem fluidundurchlässigen, vorzugsweise folienförmigen Material ausgebildet, das an seiner bzw. seinen Seitenkanten fluiddicht mit der Unterseite verbunden ist. Es kann sich bei der Oberseite auch um eine semipermeable, mikroporöse Flachmembran handeln. In diesem Fall weist die Oberseite jedoch eine geringere Permeabilität gegenüber Fluiden auf als die Unterseite, um in der Anwendung eine Verteilung zugeführter Flüssigkeit auf der Unterseite der taschenförmigen Wundauflage und damit zur Wunde hin zu gewährleisten. Im Falle, dass es sich bei Unterseite und Oberseite um dieselbe oder die gleiche semipermeablen mikroporösen Flachmembran handelt, weist die Unterseite Perforationen auf.

Als Materialien für die Unter- bzw. die Oberseite der taschenförmigen Wundauflage kommen grundsätzlich dieselben organischen Polymere in Frage, die zuvor als Polymere für die Kapillarmembranen genannt wurden und die sich zu Flachfolien bzw. Flachmembranen verarbeiten lassen. Vorzugsweise sind Unter- und/oder Oberseite der taschenförmigen Wundauflage aus Polyolefinen, Polyamiden, Polyacrylnitril, Polycarbonaten Polyester oder Sulfonpolymeren sowie daraus gewonnen Modifikationen, Blends, Mischungen oder Copolymere dieser Polymere aufgebaut. Besonders bevorzugt umfassen Unter- und Oberseite als Material Sulfonpolymere, wobei Polysulfon oder Polyethersulfon bestens geeignet sind.

Das Wundauflagesystem kann in der taschenförmigen Wundauflage auch mehrere Kapillarmembransysteme enthalten, die unterschiedliche Aufgaben im Wundversorgungssystem übernehmen können. So kann das Wundauflagesystem ein erstes Kapillarmembransystem aufweisen, über das eine Versorgung der Wunde mit beispielsweise Nährlösung erfolgen kann und ein zweites Kapillarmembransystem, über das Exsudat aus der Wunde entfernt werden kann. Wie zuvor ausgeführt wurde, kann das Wundauflagesystem auch ein Kapillarmembransystem umfassen, über das eine Oxidation erfolgen kann, d.h. über das der Wunde Sauerstoff zugeführt werden kann. Auch die Kombination mit einem weiteren Kapillarmembransystem ist möglich, über das z.B. eine Temperierung oder eine pH-Wert Regulierung erfolgen kann. Dabei sind die in den Kapillarmembransystemen enthaltenen Kapillarmembranen an die jeweilige Aufgabe des Kapillarmembransystems angepasst, wie dies bereits zuvor ausgeführt wurde, wobei sich die Kapillarmembranen der einzelnen Systeme auch hinsichtlich der sie aufbauenden Materialien unterscheiden können. Des weiteren können die Systeme auch hinsichtlich ihres Aufbaus unterschiedlich sein, beispielsweise hinsichtlich der Anzahl der in ihnen enthaltenen Kapillarmembranen, deren Abstand zueinander, der Anzahl der Versorgungsleitungen, mit denen sie verbunden sind usw..

Dabei können die jeweiligen Kapillarmembransysteme aufeinander gelegt werden. Es ist jedoch auch möglich, dass zwei unterschiedliche Kapillarmembransysteme miteinander zu einer Matte verbunden sind, wobei die Kapillarmembranen der unterschiedlichen Kapillarmembransysteme mit ihren Enden in unterschiedliche Versorgungsleitungen eingebettet sind, die vorzugsweise an entgegengesetzten Seiten der Matte angeordnet sind. Solche Matten können beispielsweise durch Verwirken von zueinander versetzt angeordneten, mäanderförmig abgelegten Kapillarmembranen erhalten werden, bei denen die U-förmigen Umlenkungen der Kapillarmembranen sich an unterschiedlichen Positionen über der Mattenbreite befinden. Durch Schneiden der jeweils außenliegenden U-förmigen Umlenkungen werden die Kapillarmembranen an jeweils nur einer Seite der Matte geöffnet und können dort in eine Versorgungsleitung eingebettet werden.

In einer Ausführungsform kann insbesondere bei kleinen Wundauflagen sich im Tascheninneren ansammelndes Exsudat über die Oberseite der taschenförmigen Wundauflage z.B. mittels eines an der Oberseite angebrachten und mit dem Tascheninneren in Fluidverbindung stehenden Absaugstutzens entfernt werden. Der Absaugstutzen kann mit einer Unterdruckeinheit verbundbar sein, um so in der Anwendung einen Unterdruck im Tascheninneren zu erzeugen.

In einer vorteilhaften Ausführungsform kann im Tascheninneren ein Drainagesystem angeordnet sein, welches zur Entfernung von Exsudat aus zu behandelnden Wunden geeignet ist. Wie zuvor erläutert, kann das Wundauflagesystem zur Entfernung von Exsudat ein eigenes Kapillarmembransystem aufweisen, das in diesem Fall das Drainagesystem darstellt. Das Drainagesystem kann vorzugsweise aber auch mindestens ein Drainagekatheter sein, der über eine an seinen Querschnitt fluiddicht angepasste Durchtrittsöffnung aus der taschenförmigen Wundauflage herausführt und mit einer Unterdruckeinheit verbindbar ist, um so in der Anwendung einen Unterdruck im Tascheninneren zu erzeugen. Bei dem mindestens einen Drainagekatheder kann es sich um ein Schlauchstück, beispielsweise aus einem Silikonmaterial, oder um ein Röhrchen handeln, das im Tascheninneren der Wundauflage angeordnet ist und über die Durchtrittsöffnung aus der Wundauflage herausführt. An dem sich im Inneren der Wundauflage befindlichen Segment des mindestens einen Drainagekatheters weist dieser vorzugsweise in seiner Wand Perforierungen auf, über die nach Anschluss des mindestens einen Drainagekatheders an eine Unterdruckeinheit z.B. Exsudat aus der Wunde bzw. aus dem Inneren der taschenförmigen Wundauflage und aus der Wunde abgesaugt werden kann. Der mindestens eine Drainagekatheter weist bevorzugt einen Innendurchmesser im Bereich von 0,1 bis 15 mm auf und eine Wandstärke im Bereich von 0,1 bis 3 mm und erstreckt sich vorzugsweise über mindestens die gesamte Länge oder Breite des Tascheninneren. Der Drainagekatheter kann auch einen nicht-kreisförmigen Querschnitt aufweisen. In diesem Fall wird als Innendurchmesser der äquivalente Durchmesser $d_D = 4A_D/U_D$ des Innenquerschnitts angesetzt mit $A_D$ als der Fläche des Innenquerschnitts des Drainagekatheters und $U_D$ als dessen Umfang.

Wie ausgeführt, kann die mindestens eine Versorgungsleitung des mindestens einen Kapillarmembransystems über eine an ihren äußeren Querschnitt angepasste Durchtrittsöffnung fluiddicht aus der taschenförmigen Wundauflage herausführt werden und ist außerhalb der taschenförmigen Wundauflage mit einer Versorgungseinheit verbindbar. Gleiches gilt bzgl. des gegebenenfalls vorhandenen Drainagekatheters, der über eine an den äußeren Querschnitt des Drainagekatheters angepasste Durchtrittsöffnung aus der Wundauflage herausgeführt wird. Die jeweilige Durchtrittsöffnung kann sich in der Oberseite der taschenförmigen Wundauflage befinden oder im Bereich der Verbindung zwischen Unter- und Oberseite der Wundauflage. Bei Durchtrittsöffnungen, die in der Oberseite der Wundauflage angebracht sind, ist es erforderlich, dass die Durchtrittsöffnungen zwischen der Versorgungsleitung bzw. dem Drainagekatheter und dem die Oberseite ausbildenden folienförmigen Material beispielsweise mittels Silikon abgedichtet sind. Bei Anordnung der Durchtrittsöffnungen im Bereich der Verbindung zwischen Unter- und Oberseite der Wundauflage kann die Abdichtung gleichzeitig mit dem Verschweißen oder Verkleben von Unter- und Oberseite erfolgen. In Bezug auf die Handhabung ist es zweckmäßig, wenn die Versorgungs- und ggf. vorhandenen Drainageleitungen zur gleichen Seite der Wundauflage aus der taschenförmigen Wundauflage herausführen.

Für die Charakterisierung der Eigenschaften der im Wundauflagesystem eingesetzten Kapillarmembranen bzw. Flachmembranen werden die folgenden Messmethoden zu Grunde gelegt:

Transmembranfluss (Wasserpermeabilität) für Kapillarmembranen:

Aus den zu prüfenden Kapillarmembranen wird eine Prüfzelle mit definierter Kapillarmembranzahl und Länge gefertigt. Die Kapillarmembranen werden dafür beidseitig an ihren Enden in ein Polyurethanharz eingebettet. Nach dem Aushärten des Harzes werden die Einbettungen auf eine Länge von ca. 30 mm geschnitten, wobei die Lumina der Kapillarmembranen durch den Schnitt geöffnet werden. Die Kapillarlumina in den Einbettungen müssen auf Durchgängigkeit überprüft werden. Die freie Länge der Kapillarmembranen zwischen den Einbettungen beträgt üblicherweise 120 +/- 10 mm. Die Anzahl der Kapillarmembranen ist so zu bemessen, dass unter Berücksichtigung der freien Länge und des Innendurchmessers der Kapillarmembranen eine Filtrationsfläche von ca. 30 cm$^2$ in der Prüfzelle bereitgestellt wird.

Die Prüfzelle wird mit in eine Prüfapparatur eingebunden und mit auf 25°C temperiertem ultrafiltriertem und vollentsalztem Wasser bei einem definiertem Prüfdruck (ca. 0,4 bar) durchströmt. Die während einer Messzeit von 2 min erhaltene filtrierte Wassermenge, d.h. das während der Messung erzeugte Permeat wird gravimetrisch oder volumetrisch erfasst. Vor Beginn der Messung muss die Anlage luftfrei gespült werden. Zur Bestimmung des TMF werden in der Prüfapparatur der Eingangs- und Ausgangsdruck an der Prüfzelle gemessen. Die Messung wird bei 25°C durchgeführt.

Der Transmembranfluss TMF wird nach der Formel (I)

$$TMF = \frac{V_W}{\Delta t \cdot A_M \cdot \Delta p} \quad \left[\frac{ml}{cm^2 \cdot min \cdot bar}\right] \quad \quad (I)$$

ermittelt. Hierbei sind:

$V_W$ = durch die Membranprobe während der Messzeit hindurch geströmte Wasservolumen [ml]
$\Delta t$ = Messzeit [min]
AM = durchströmte Fläche der Membranprobe (üblicherweise 30 cm$^2$)
$\Delta p$ = eingestellter Druck während der Messung [bar]

Permeabilität für Wasser der Unterseite der taschenförmigen Wundauflage:

Aus dem zu prüfenden flächigen Material der Unterseite der taschenförmigen Wundauflage werden scheibenförmige Proben ausgestanzt und in einen geeigneten Probenhalter am Umfang fluiddicht so eingespannt, dass eine freie Messfläche von 17,35 cm$^2$ resultiert. Der Probenhalter befindet sich in einem Gehäuse, das von Wasser druckbeaufschlagt durchströmt werden kann. Die eingespannte Probe wird dann von auf 25°C temperiertem, vollentsalztem Wasser unter einem definierten Druck zwischen 0,1 und 0,2 bar durchströmt. Es wird das während einer Messzeit von 60 s durch die Probe hindurch geströmte Wasservolumen gravimetrisch oder volumetrisch ermittelt.

Die Permeabilität für Wasser $TMF_W$ wird nach der Formel (II)

$$TMF_W = \frac{V_W}{\Delta t \cdot A_M \cdot \Delta p} \quad \left[\frac{ml}{cm^2 \cdot min \cdot bar}\right] \quad \quad (II)$$

ermittelt. Hierbei sind:

$V_W$ = durch die Probe während der Messzeit hindurch geströmte Wasservolumen [ml]
$\Delta t$ = Messzeit [min]
AM = durchströmte Fläche der Probe (17,35 cm$^2$)
$\Delta p$ = eingestellter Druck während der Messung [bar]

Die Erfindung wird anhand der folgenden Figuren näher erläutert, wobei durch die Figuren der Umfang der Erfindung nicht eingeschränkt wird:

Es zeigen:

Fig. 1: schematisch einen Querschnitt durch ein taschenförmiges Wundauflagensystem gemäß der Erfindung

Fig. 2: das in Figur 1 im Querschnitt dargestellte Wundauflagesystem in einem Schnitt A - A.

Fig. 3: das Wundauflagesystem der Figuren 1 und 2 in einer Draufsicht auf die untere Seite der Unterseite der taschenförmigen Wundauflage.

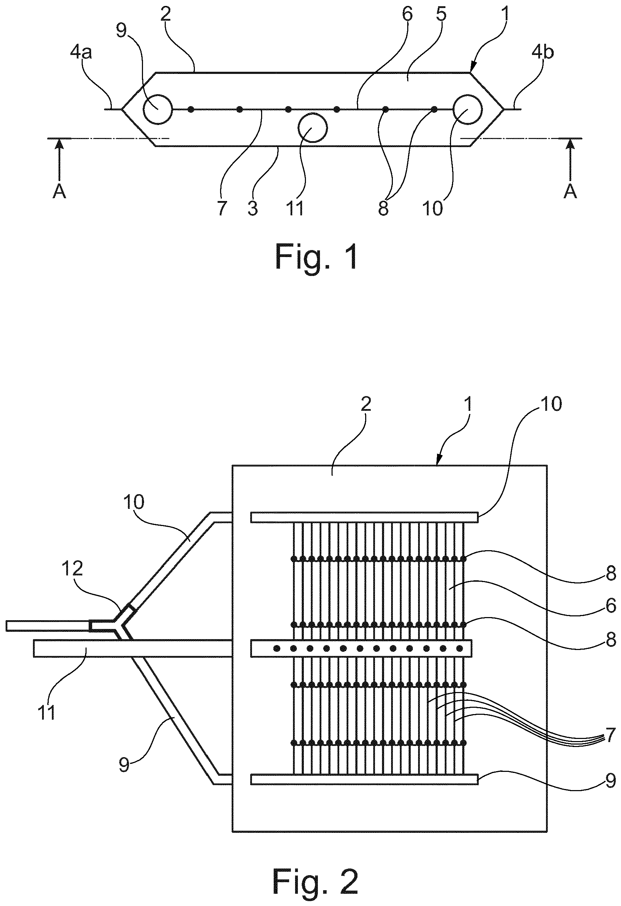

Figur 1 zeigt schematisch einen Querschnitt durch ein taschenförmiges Wundauflagensystem mit einer taschenförmigen Wundauflage 1, welche eine Oberseite 2 und eine Unterseite 3 aufweist, die an ihrem Rand 4a, 4b z.B. miteinander verschweißt sind, wodurch ein geschlossenes Tascheninneres 5 entsteht. Im Tascheninneren 5 ist ein flächenförmiges Kapillarmembransystem 6 angeordnet, welches Kapillarmembranen 7 umfasst, die über zueinander parallel verlaufende Verbindungselemente 8 vorzugsweise in Gestalt von Multifilamentgarnen miteinander verbunden und zueinander auf Abstand gehalten sind.

Die Kapillarmembranen 7 münden im vorliegenden Fall mit ihren gegenüberliegenden Enden in Versorgungsleitungen 9,10, so dass durch die Versorgungsleitungen 9,10 und das Kapillarmembransystem 6 Flüssigkeiten, Medien, Gase und/oder andere Stoffe hindurchleitbar sind. Die Versorgungsleitungen 9, 10 werden durch die Oberseite 2 der taschenförmigen Wundauflage 1 herausgeführt (hier nicht dargestellt).

Unterhalb des flächenförmigen Kapillarmembransystems 6 ist ein Drainageschlauch 11 angeordnet, über den z.B. sich in der Wunde ansammelndes Exsudat entfernt werden kann.

Figur 2 zeigt das in Figur 1 dargestellte Wundauflagesystem in einem Querschnitt entlang der Linie A - A. Im Prinzip handelt es sich um eine Draufsicht, von einer Position oberhalb der Unterseite 3 der taschenförmigen Wundlauflage 1 in Richtung der Oberseite 2 der taschenförmigen Wundlauflage 1. Unterhalb der Oberseite 2 , d.h. wie in Figur 1 dargestellt, zwischen Unterseite 3 und Oberseite 2 ist das flächenförmige Kapillarmembransystem 6 angeordnet, das aus zueinander parallelen Kapillarmembranen 7 aufgebaut ist, die über die Verbindungselemente 8 miteinander verbunden und zueinander auf Abstand gehalten sind. Die Kapillarmembranen 7 sind mit ihren gegenüberliegenden Enden in die Versorgungsleitungen 9, 10 eingebettet, so dass durch die Versorgungsleitungen 9,10 und das Kapillarmembransystem 6 Flüssigkeiten, Medien, Gase und/oder andere Stoffe hindurchleitbar sind. Die Versorgungsleitungen 9,10 werden durch die Oberseite 2 der taschenförmigen Wundauflage 1 durch entsprechend angepasste Öffnungen in der Oberseite 2 aus der taschenförmigen Wundauflage 1 herausgeführt und im vorliegenden Beispiel über einen $\gamma$-Verbinder 12 außerhalb der taschenförmigen Wundauflage 1 zusammengeführt. Im vorliegenden Fall wird also das flächenförmige Kapillarmembransystem 6 im Dead-end Modus betrieben, d.h. ein über die Versorgungsleitungen 9, 10 zugeführtes Medium wird in das Kapillarmembransystem 6 eingeleitet und tritt vollständig über die Wände der Kapillarmembranen 7 in das Tascheninnere ein.

In der Figur 2 ist auch der Drainageschlauch 11 dargestellt, der unterhalb des Kapillarmembransystems 6 angeordnet ist. Der Drainageschlauch weist in seiner Wand Perforationen auf, so dass z.B. sich in der Wunde ansammelndes Exsudat über den Drainageschlauch angesaugt und so aus der Wunde entfernt werden kann. Der Drainageschlauch 11 wird ebenfalls über eine entsprechend angepasste Öffnung in der Oberseite 2 aus der taschenförmigen Wundauflage 1 herausgeführt und ist z.B. mit einer (nicht dargestellten) Unterdruckeinheit verbindbar.

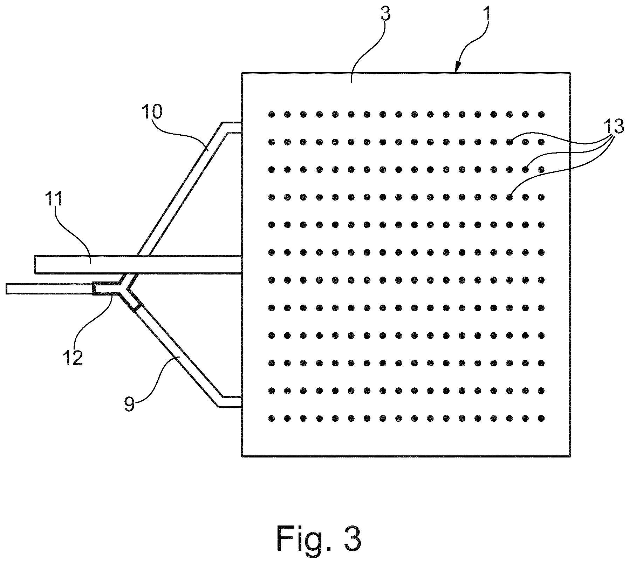

Figur 3 zeigt das Wundauflagesystem der Figuren 1 und 2 in einer Draufsicht auf die untere Seite der Unterseite 3 der taschenförmigen Wundauflage. Diese untere Seite der Unterseite der taschenförmigen Wundauflage wird in der Anwendung auf die Wunde aufgelegt.

Im dargestellten Beispiel ist eine Unterseite 3 gezeigt, die Perforationen 13 in ihrer Fläche aufweist, wobei die Perforationen über die Fläche gleichmäßig verteilt und in Reihen zueinander angeordnet sind. Natürlich sind auch andere Verteilungen der Perforationen denkbar. Über die Perforationen in der Unterseite der taschenförmigen Wundauflage können z.B. mittels des in der Wundauflage enthaltenen Kapillarmembransystems in das Tascheninnere eingetragene Nährlösungen gleichmäßig der Wunde zugeführt werden. Ebenso ist es möglich, über die Perforationen Exsudat aus der Wunde abzusaugen und damit das Exsudat aus der Wunde zu entfernen.

In Figur 3 sind ebenfalls die Versorgungsleitungen 9, 10 dargestellt, die aus der (hier nicht dargestellten) Oberseite der taschenförmigen Wundauflage 1 herausgeführt und über den $\gamma$-Verbinder 12 außerhalb der taschenförmigen Wundauflage 1 zusammengeführt sind. Ebenso ist der Drainageschlauch 11 dargestellt, über den z.B. sich in der Wunde ansammelndes Exsudat entfernt werden kann und der wie die Versorgungsleitungen 9, 10 aus der Oberseite der taschenförmigen Wundauflage 1 herausgeführt wird, wie in Figur 1 gezeigt.

**Patentansprüche**

1.   Wundauflagesystem zum Einbringen in eine Wunde umfassend mindestens ein flächenförmig ausgebildetes Kapillarmembransystem [6], **dadurch gekennzeichnet, dass** das mindestens eine flächenförmig ausgebildete Kapillarmembransystem [6] in einer taschenförmigen Wundauflage [1] angeordnet ist, die an ihrem äußeren Rand [4a; 4b] geschlossen ist und eine Oberseite [2], eine Unterseite [3] und ein Tascheninneres [5] aufweist,

- wobei die Unterseite [3] und die Oberseite [2] jeweils aus einem flächigen Material ausgebildet sind und die Unterseite [3] permeabel für Fluide ist,
- wobei das mindestens eine flächenförmig ausgebildete Kapillarmembransystem [6] im Tascheninneren [5] angeordnet und mit mindestens einer Versorgungsleitung [9; 10] verbunden ist, so dass durch die Versorgungsleitung [9; 10] und das mindestens eine Kapillarmembransystem [6] Flüssigkeiten, Medien, Gase und/oder andere Stoffe durchleitbar sind, und
- wobei die mindestens eine Versorgungsleitung [9; 10] außerhalb der taschenförmigen Wundauflage [1] mit einer Einheit zur Versorgung bzw. Entsorgung verbindbar ist.

2. Taschenförmiges Wundauflagesystem nach Anspruch 1, **dadurch gekennzeichnet, dass** die Unterseite [3] der taschenförmigen Wundauflage [1] aus einem vliesförmigen, flächigen Material oder einer semipermeablen, mikroporösen Flachmembran ausgebildet ist.

3. Taschenförmiges Wundauflagesystem nach Anspruch 2, **dadurch gekennzeichnet, dass** die Unterseite [3] der taschenförmigen Wundauflage [1] aus einer semipermeablen, mikroporösen Flachmembran ausgebildet ist.

4. Taschenförmiges Wundauflagesystem nach einem oder mehreren der Ansprüche 1 bis 3, **dadurch gekennzeichnet, dass** die Unterseite [3] der taschenförmigen Wundauflage [1] eine Permeabilität für Wasser von mindestens 0,01 ml/(min·cm$^2$·bar) aufweist.

5. Taschenförmiges Wundauflagesystem nach einem oder mehreren der Ansprüche 1 bis 4, **dadurch gekennzeichnet, dass** die Unterseite [3] der taschenförmigen Wundauflage [1] Öffnungen aufweist.

6. Taschenförmiges Wundauflagesystem nach einem oder mehreren der Ansprüche 1 bis 5, **dadurch gekennzeichnet, dass** sich die Verbindung des mindestens einen flächenförmig ausgebildeten Kapillarmembransystems [6] mit der mindestens einen Versorgungsleitung [9; 10] im Tascheninneren [5] befindet und die mindestens eine Versorgungsleitung [9; 10] über eine an ihren äußeren Querschnitt fluiddicht angepasste Durchtrittsöffnung aus der taschenförmigen Wundauflage [1] herausführt.

7. Taschenförmiges Wundauflagesystem nach einem oder mehreren der Ansprüche 1 bis 5, **dadurch gekennzeichnet, dass** die Verbindung des mindestens einen flächenförmig ausgebildeten Kapillarmembransystems [6] mit der mindestens einen Versorgungsleitung [9; 10] außerhalb der taschenförmigen Wundauflage [1] auf der Oberseite [2] angeordnet ist und das mindestens eine flächenförmig ausgebildete Kapillarmembransystem [6] zur Verbindung mit der mindestens einen Versorgungsleitung [9;10] über eine fluiddicht angepasste Durchtrittsöffnung aus der taschenförmigen Wundauflage [1] herausführt.

8. Taschenförmiges Wundauflagesystem nach einem oder mehreren der Ansprüche 1 bis 7, **dadurch gekennzeichnet, dass** im Tascheninneren [5] ein Drainagesystem [11] angeordnet ist, welches zur Entfernung von Exsudat aus zu behandelnden Wunden geeignet ist.

9. Taschenförmiges Wundauflagesystem nach Anspruch 8, **dadurch gekennzeichnet, dass** das Drainagesystem [11] ein Drainagekatheter ist, der über eine an seinen Querschnitt fluiddicht angepasste Durchtrittsöffnung aus der taschenförmigen Wundauflage [1] herausführt und mit einer Unterdruckeinheit verbindbar ist.

10. Taschenförmiges Wundauflagesystem nach einem oder mehreren der Ansprüche 1 bis 9, **dadurch gekennzeichnet, dass** das Kapillarmembransystem [6] in Form einer Matte aus parallel zueinander angeordneten Kapillarmembranen [7] ausgebildet ist, wobei die Kapillarmembranen [7] in der Matte mittels zueinander beabstandeter und zueinander parallel verlaufender Verbindungselemente [8] miteinander verbunden und durch die Verbindungselemente [8] zueinander auf Abstand gehalten sind.

11. Taschenförmiges Wundauflagesystem nach einem oder mehreren der Ansprüche 1 bis 10, **dadurch gekennzeichnet, dass** die Kapillarmembranen [7] mit mindestens einem ihrer Enden in der mindestens einen Versorgungsleitung [9; 10] eingebettet sind.

12. Taschenförmiges Wundauflagesystem nach Anspruch 11, **dadurch gekennzeichnet, dass** das Kapillarmembransystem [6] mit zwei Versorgungsleitungen [9;10] verbunden ist, wobei die Kapillarmembranen [7] mit ihren gegenüberliegenden Enden in jeweils eine Versorgungsleitung [9; 10] eingebettet sind.

13. Taschenförmiges Wundauflagesystem nach einem oder mehreren der Ansprüche 1 bis 12, **dadurch gekennzeichnet, dass** die Kapillarmembranen [7] einen Transmembranfluss für Wasser im Bereich von 0,01 bis 50 ml/(min·cm$^2$·bar) aufweisen.

**Claims**

1. A wound dressing system for introduction into a wound, comprising at least one uniplanar capillary membrane system [6], **characterized in that** the at least one uniplanar capillary membrane system [6] is arranged in a bag- type wound dressing [1], which is closed on its external edge [4a, 4b] and has an upper side [2], a lower side [3], and a bag interior [5],

    - wherein the lower side [3] and the upper side [2] are each formed from a flat material and the lower side [3] is permeable to fluids,
    - wherein the at least one uniplanar capillary membrane system [6] is arranged in the bag interior [5] and is connected to at least one supply line [9, 10] such that fluids, media, gases, and/or other substances can be supplied through the supply line [9, 10] and the at least one capillary membrane system [6], and
    - wherein the at least one supply line [9, 10] can be connected to a unit for provision or removal outside of the bag-type wound dressing [1]

2. The bag-type wound dressing system according to claim 1, **characterized in that** the lower side [3] of the bag-type wound dressing [1] is formed from a nonwoven, flat material or a semi-permeable, microporous flat membrane.

3. The bag-type wound dressing system according to claim 2, **characterized in that** the lower side [3] of the bag-type wound dressing [1] is formed from a semi-permeable, microporous flat membrane.

4. The bag-type wound dressing system according to any of claims 1 to 3, **characterized in that** the lower side [3] of the bag-type wound dressing [1] has a permeability to water of at least 0.01 cm$^3$/(min·cm$^2$·0.1 MPa) (0.01 mL/(min·cm$^2$·bar)).

5. The bag-type wound dressing system according to any of claims 1 to 4, **characterized in that** the lower side [3] of the bag-type wound dressing [1] has openings.

6. The bag-type wound dressing system according to any of claims 1 to 5, **characterized in that** the connection of at least one uniplanar capillary membrane system [6] to the at least one supply line [9, 10] is located in the bag interior [5], and the at least one supply line [9, 10] exits from the bag-type wound dressing [1] via a passage that is adapted to be sealed against fluids at its external cross-section.

7. The bag-type wound dressing system according to any of claims 1 to 5, **characterized in that** the connection of at least one uniplanar capillary membrane system [6] to the at least one supply line [9, 10] is arranged outside the bag-type wound dressing [1] on the upper side [2], and the at least one uniplanar capillary membrane system [6] for connecting to the at least one supply line [9, 10] exits from the bag-type wound dressing [1] via a passage that is adapted to be sealed against fluids.

8. The bag-type wound dressing system according to any of claims 1 to 7, **characterized in that** a drainage system [11], which is suitable for removing exudate from wounds to be treated, is arranged in the bag interior [5].

9. The bag-type wound dressing system according to claim 8, **characterized in that** the drainage system [11] is a drainage catheter, which exits the bag-type wound dressing [1] via a passage adapted to be sealed against fluids at its cross-section and which can be connected to a vacuum unit.

10. The bag-type wound dressing system according to any of claims 1 to 9, **characterized in that** the capillary membrane system [6] is formed as a pad made of capillary membranes [7] arranged parallel to one another, wherein the capillary membranes [7] are connected to one another by means of connecting elements [8] in the pad, which extend in intervals to one another and parallel to one another, and are maintained in intervals to one another by means of the connecting elements [8].

11. The bag-type wound dressing system according to any of claims 1 to 10, **characterized in that** the capillary membranes [7] are embedded in the at least one supply line [9, 10] with at least one of their ends.

12. The bag-type wound dressing system according to claim 11, **characterized in that** the capillary membrane system [6] is connected to two supply lines [9, 10], wherein the capillary membranes [7] are each embedded in a supply line [9, 10] with their opposite ends.

13. The bag-type wound dressing system according to any of claims 1 to 12, **characterized in that** the capillary membranes [7] have a transmembrane water flow ranging from 0.01 to 50 $cm^3$/(min·$cm^2$·0.1 MPa) (from 0.01 to 50 mL(min·$cm^2$·bar)).

**Revendications**

1. Système de pansement à appliquer sur une plaie, comprenant au moins un système de membrane capillaire en forme de feuille [6], **caractérisé en ce qu'au** moins un système de membrane capillaire en forme de feuille [6] est disposé dans un pansement en forme de poche [1], qui est fermé au niveau de son bord externe [4a ; 4b] et présente un côté supérieur [2], un côté inférieur [3] et un espace interne de poche [5],

   - le côté inférieur [3] et le côté supérieur [2] étant formés respectivement d'un matériau en feuille et le côté inférieur [3] étant perméable aux fluides,
   - au moins un système de membrane capillaire en forme de feuille [6] étant agencé dans l'espace interne de poche [5] et lié à au moins une conduite d'alimentation [9 ; 10], de sorte que des fluides, des liquides, des gaz et/ou d'autres matières puissent être guidés à travers la conduite d'alimentation [9 ; 10] et au moins un système de membrane capillaire [6] et
   - au moins une conduite d'alimentation [9 ; 10] pouvant être reliée en dehors du pansement en forme de poche [1] avec une unité pour l'alimentation ou l'élimination.

2. Système de pansement en forme de poche selon la revendication 1, **caractérisé en ce que** le côté inférieur [3] du pansement en forme de poche [1] est formé en un matériau en feuille non tissée ou une membrane plate microporeuse semi-perméable.

3. Système de pansement en forme de poche selon la revendication 2, **caractérisé en ce que** le côté inférieur [3] du pansement en forme de poche [1] est formé en une membrane plate microporeuse semi-perméable.

4. Système de pansement en forme de poche selon une ou plusieurs des revendications 1 à 3, **caractérisé en ce que** le côté inférieur [3] du pansement en forme de poche [1] présente une perméabilité à l'eau d'au moins 0,01 $cm^3$/(min·$cm^2$·0,1 MPa) (0,01 ml/(min·$cm^2$·bar)).

5. Système de pansement en forme de poche selon une ou plusieurs des revendications 1 à 4, **caractérisé en ce que** le côté inférieur [3] du pansement en forme de poche [1] présente des ouvertures.

6. Système de pansement en forme de poche selon une ou plusieurs des revendications 1 à 5, **caractérisé en ce que** la liaison entre au moins un système de membrane capillaire en forme de feuille [6] et au moins une conduite d'alimentation [9 ; 10] se trouve dans l'espace interne de la poche [5] et au moins une conduite d'alimentation [9 ; 10] fait saillie hors du pansement en forme de poche [1] via une ouverture de passage adaptée à sa section transversale externe étanche aux fluides.

7. Système de pansement en forme de poche selon une ou plusieurs des revendications 1 à 5, **caractérisé en ce que** la liaison entre au moins un système de membrane capillaire en forme de feuille [6] et au moins une conduite d'alimentation [9 ; 10] est disposée en dehors du pansement en forme de poche [1] sur le côté supérieur [2] et au moins un système de membrane capillaire en forme de feuille [6] fait saillie hors du pansement en forme de poche [1] via une ouverture de passage adaptée étanche aux fluides pour une liaison avec au moins une conduite d'alimentation [9 ; 10].

8. Système de pansement en forme de poche selon une ou plusieurs des revendications 1 à 7, **caractérisé en ce**

**EP 3 068 455 B1**

**que** dans l'espace interne de poche [5] se trouve un système de drainage [11], destiné à l'élimination d'exsudat de la plaie à traiter.

9. Système de pansement en forme de poche selon la revendication 8, **caractérisé en ce que** le système de drainage [11] est un cathéter de drainage, qui fait saillie par le biais d'une ouverture de passage adaptée à sa section transversale étanche aux fluides hors du pansement en forme de poche [1] et peut être relié à une unité de dépression.

10. Système de pansement en forme de poche selon une ou plusieurs des revendications 1 à 9, **caractérisé en ce que** le système de membrane capillaire [6] est formé sous la forme d'une natte de membranes capillaires disposées de façon parallèles les unes aux autres [7], les membranes capillaires [7] dans la natte étant reliées les unes aux autres par le biais d'éléments de liaison [8] espacés et parallèles entre eux et espacées les unes des autres à une certaine distance par les éléments de liaison [8].

11. Système de pansement en forme de poche selon une ou plusieurs des revendications 1 à 10, **caractérisé en ce que** les membranes capillaires [7] sont intégrées avec au moins une de leurs extrémités dans au moins une conduite d'alimentation [9 ; 10].

12. Système de pansement en forme de poche selon la revendication 11, **caractérisé en ce que** le système de membrane capillaire [6] est relié avec deux conduites d'alimentation [9 ;10], les membranes capillaires [7] étant intégrées respectivement dans une conduite d'alimentation [9 ; 10] avec leurs extrémités opposées.

13. Système de pansement en forme de poche selon une ou plusieurs des revendications 1 à 12, **caractérisé en ce que** les membranes capillaires [7] présentent un flux trans-membranaire pour l'eau dans la plage de 0,01 à 50 $cm^3$/(min·$cm^2$·0,1 MPa) (de 0,01 à 50 ml/(min·$cm^2$·bar)).

Fig. 1

Fig. 2

Fig. 3

EP 3 068 455 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102006042732
- EP 1144096 A
- EP 0299381 A
- DE 2833493 A
- DE 3839567
- DE 4308850
- EP 0442147 A

15